# EUROPEAN PATENT SPECIFICATION

(11) **EP 0 528 886 B1**
(45) Date of publication and mention of the grant of the patent: **23.10.1996**
(21) Application number: 91909160.3
(22) Date of filing: 06.05.1991
(51) Int. Cl.: C07K 4/12, C12N 5/08, A61K 39/395, A61K 39/00, A61K 38/00, G01N 33/564, G01N 33/577, G01N 33/68

(54) **BETA CELL ANTIGEN**
BETA-ZELL-ANTIGEN
ANTIGENE DE CELLULE BETA

(30) Priority: 04.05.1990 NL 9001083
(43) Date of publication of application: 03.03.1993
(73) Proprietor: Rijksuniversiteit te Leiden, NL-2312 AV Leiden (NL)
(72) Inventor: ROEP, Bart, Otto Rijksuniversiteit te Leiden, NL-2312 AV Leiden (NL); DE VRIES, Rene, Rudolf, Pieter, NL-2312 AV Leiden (NL); HUTTON, John, Charles Rijksuniversiteit te Leiden, NL-2312 AV Leiden (NL)
(74) Representative: Smulders, Theodorus A.H.J., Ir.
(86) International application number: NL9100077
(87) International publication number: WO9117186

(56) References cited:
- PROCEEDINGS OF THE NATL. ACADEMY OF SCIENCES, vol. 86, no. 20, October 1989, Immunology, (US); K. HASKINS et al., pp. 8000-8004
- EUROPEAN JOURNAL OF IMMUNOLOGY, vol. 19, 1989, VCH Verlagsges. mbH, Weinheim (DE); E. VAN VLIET et al., pp. 213-216
- NATURE, vol. 345, 14 June 1989, London (GB); B.O. ROEP et al., pp. 632-634

## Description

This invention relates to an antigen of beta cells, which antigen comprises an epitope specifically recognised by T cells involved in the pathological process of type 1 diabetes mellitus.

Type 1 (insulin-dependent) diabetes mellitus is a disease having a prevalence of about 0.2% in northwest Europe. The incidence has doubled the past 20 years. In general, the disease manifests itself before the nineteenth year of life. Life expectancy is about 10 years below that of the healthy population. It is an important health problem in the Western population, partly also because the disease is connected with a large number of complications, such as blindness, cardiovascular diseases, kidney insufficiency and neuritis. Type 1 diabetes is caused by the irreversible loss of the insulin producing cells (beta cells) in the pancreatic islets of Langerhans owing to an inflammation process, the cause of which is unknown. As a result of this loss of the beta cells the patient depends on exogenous insulin (administered from the outside) for the rest of his life. Pancreas transplantation seems hardly useful as long as the process leading to the destruction of beta cells has not been defined and cannot be stopped.

The destruction of the beta cells is probably the result of an autoimmune process. Indicative of this is, inter alia, the infiltration of the islets of Langerhans by mononuclear cells including many T lymphocytes (insulitis) at the moment of diagnosis. Another indication of a relation between the immune system and type 1 diabetes is the association of specific genetic markers of the HLA (human leucocyte associated antigens) system, the name for the human MHC (major histocompatibility complex), which plays a central role in the regulation of the immune response. Especially HLA-DR3 and/or -DR4 positive individuals have an increased relative risk of developing the disease, while certain HLA-DQ alleles seem to be associated with resistance.

A similar protection encoded by the MHC is also found in the mouse model for type 1 diabetes, the NOD mouse. This mouse strain is normally I-E negative (I-E is the equivalent in mice of HLA-DQ in human beings) and spontaneously develops diabetes in a part of the population.

As early as 15 years ago islet cell autoantibodies (ICA's) were found for the first time in serum of type 1 diabetes patients. It has been shown later that these ICA's can often be detected in the serum years before the clinical manifestation of the disease. But the islet cell determinant against which these antibodies are directed has still not been identified. As the only structure a 64 kD protein has been precipitated by means of serum of (pre)diabetes patients. This protein has recently been defined as the neuronal enzyme glutamic acid decarboxylase. ICA's seem to be disease (type 1 diabetes) and target cell (beta cell) specific so that these antibodies have a predictive value in respect of the development of type 1 diabetes. This creates the possibility of identifying persons before the disease manifests itself clinically so that in that case immunotherapy can lead to prevention of total beta cell destruction, and hence of diabetes.

It is unlikely, however, that ICA's play a role in the pathogenesis of type 1 diabetes. Studies with test animal models of this disease (NOD mice, BB rats) have shown that T lymphocytes (both of T helper type CD4⁺ and of cytotoxic T cell type CD8⁺) can transfer the disease to healthy recipients. The CD4⁺ T cells responsible for this transfer were cloned and proved to be specific for islet cell antigens. The histology of pancreases of mice injected with the pathogenic T cell clones showed the characteristic insulitis previous to the development of diabetes. The expression of I-E is associated with deletion during the ontogeny of T cells using a specific T cell receptor for the recognition of antigen.

The best indications of a role for T cells in the pathogenesis of type 1 diabetes in human beings come from studies of pancreas transplantations between identical twins and HLA identical brothers or sisters, one of the two being a type 1 diabetes patient. When a pancreas transplant was transplanted from the healthy donor to the patient, no rejection of the transplant took place, but yet the transplanted islets were not capable of making the patient exogenous insulin independent. Histology of the transplant again showed the insulitis characteristic of diabetes, probably the result of a flare-up of the "auto"immune response against beta cell determinants. This insulitis did not go with an increase in the ICA titres, which speaks against ICA as an effector mechanism in type 1 diabetes. If these autoreactive T cells responsible for beta cell destruction as well as the structures against which these T cells are directed would be characterised, strategies for immunotherapy and prevention could be developed.

The inventors have now isolated T cells from the blood of a recently diagnosed type 1 diabetes patient which are reactive against a beta tumour cell line membrane preparation of rats. These T cell clones also react against islet preparations of dogs and a suspension of human fetal pancreas cells so that it is suggested that the antigen has been preserved in the evolution and is not rat- or tumour-specific. With these T cell clones a search was then conducted on a subcellular level for a determinant of the beta cell which is recognised by the T cell clones. By means of a discontinuous density gradient different fractions were generated which were characterised by means of specific marker proteins. Thus four fractions were defined: pellet (lysosomes, endoplasmic reticulum, mitochondria), plasma membrane, insulin secretory granules and cytosol. These fractions were presented as antigen to the panel of T cell clones. Two of the clones proved to recognise a determinant in the insulin secretory granules (ISG). The reactivity of these two clones (IC6 and IC10) with the other fractions could be attributed to contamination of these fractions with ISG. The granules were then subjected to a further fractionation in granule membrane and granule matrix. The T cell reactivity was directed against the granule membrane fraction and remained intact after a series of extractions aimed at removing extrinsic proteins. This indicates that the antigen is an intrinsic granule membrane protein. Subsequently, the molecular weight of the antigen recognised by clone IC6 was determined by means of separation of protein on the basis of molecular weight by means of SDS/polyacrylamide gel electrophoresis and electroelution of proteins from the gel fractions so generated. The antigen proved to have a molecular weight of about 38 kD. As a granule membrane protein this 38 kD protein will be temporarily exposed on the plasma membrane during insulin release by means of exocytosis and will then be available for components of the immune system. It is therefore plausible that expression of said target structures correlates with the function activity of beta cells, i.e. insulin production and secretion. This could account for the earlier described relation between beta cell activity and the development of type 1 diabetes.

Characterisation of T cell clone 1C6 has demonstrated that the variable part of the β-chain of the T cell receptor, which is involved in the recognition of the 38 kD antigen (TCR-Vβ) belongs to the TCR-Vβ19 family.

Follow-up studies have shown that the T cell response in newly diagnosed diabetes patients against the 38 kD beta cell antigen is not an isolated phenomenon. In these follow-up studies several T cell stimulation cycles with the beta cell antigen were carried out, followed by a lymphocyte proliferation experiment. Fourteen of the nineteen patients tested exhibited a significantly increased reaction to a β-cell preparation enriched for 38 kD protein, as compared with only two of the sixteen healthy controls. In 81% of the reacting patients a clear T cell response against a purified 38 kD preparation could be detected, while neither of the two responsive controls was able to recognize this preparation. These data make it very likely that a T cell response against the 38 kD antigen is disease-associated.

An antigen recognised by T cells isolated from a type 1 diabetes patient has not been identified before. It is plausible that these T cells (CD4⁺ T cells) have the potency as effector mechanism to lead to beta cell destruction. This is supported by the results from test animal models for both diabetes mellitus and a series of other autoimmune diseases such as rheumatoid arthritis and multiple sclerosis. Recently, the identification of autoreactive T cells and autoantigens in such models has resulted in the development of strategies for therapy or prevention of the autoimmune disorder.

The identification of the 38 kD beta cell antigen creates the possibility of characterising diabetes related T cell responses. On the basis of the structure on the antigen recognised by the T cells (the antigen determinant or epitope) and the structures on the T cell receptors of the clones involved in this recognition, strategies for, e.g., diagnostics, tolerance induction and specific immunotherapy of type 1 diabetes can be developed. Assuming that, by analogy with the above test animal models, a uniform set of T lymphocytes is involved in the pathological process, it is even possible to intervene in the autoimmune response by means of antibodies against the T cell receptors of these T cells or by means of vaccination with the T cells or with synthetic peptides of these T cell receptors. However, intervention in the pathological process is only possible with a timely detection of the autoimmune response. The identification according to the invention of the 38 kD autoantigen on the insulin producing beta cells therefore has implications for uses both in the field of diagnostics and in the therapeutic field. The 38 kD antigen could be used itself, e.g., to detect the autoimmune response against this beta cell structure, even before the disease manifests itself. There are indications there are antibodies present in sera of (pre)diabetes patients, which react with an intracellular 38 kD membrane protein. For the detection of such (auto)antibodies an ELISA technique could be used in which, by means of the 38 kD protein, sera of children optionally selected on the basis of sensitivity to the development of the disease are screened for reactive autoantibodies. This would mean an appreciable improvement over the present technique using very thin cryocoupes of an optimally preserved pancreas as a substrate for autoantibodies. At present the availability of such a suitable pancreas forms one of the bottlenecks in diabetes research and diagnostics. Moreover, the incubation conditions of the present diagnostic serology are so susceptible and time-consuming that a simple ELISA has considerable practical advantages. Standardisation of the protocol gives the possibility of mutually exchanging results without the occurrence of the present differences in results between the different laboratories in the world.

It is thus possible according to the invention to obtain, by means of the 38 kD beta cell antigen through the identification of an autoreactive T cell response, a better and earlier indication of beta cell destruction, e.g., in children at increased risk or having already reached a prestage of diabetes, before the clinical manifestation of the disease.

A suitable alternative for a diagnostic technique with the 38 kD antigen itself is a diagnostic technique using antibodies which specifically recognise the beta cell reactive T cells. Suitable therefor are, in particular, monoclonal antibodies against the autoreactive T cell receptor.

Moreover, using unique specific sequences of the T cell receptors on beta cell reactive T cells, a protocol can be drafted to trace such cells and then deactivate or eliminate them. Particularly suitable therefor would be a treatment with monoclonal antibodies against the autoreactive T cell receptor. A vaccination treatment or a treatment for inducing tolerance by means of synthetic peptides derived from the specific T cell receptor is a suitable alternative.

Another possibility is the generation by means of the 38 kD protein of specific T cell lines which, after an in vitro treatment, can be injected back into the patient according to a T cell vaccination protocol, as that of Cohen et al. In models of autoimmune diseases such an approach has already led to prevention of the disease or even cure of the test animal.

The invention therefore provides a beta cell antigen consisting of an intrinsic transmembrane protein of insulin secretory granules of beta cells lying in pancreatic islets of Langerhans, which protein contains an epitope recognised by T cell clone 1C6. This (human) T cell clone 1C6, which for antigen recognition uses a gene product of the TCR-Vβ19 family, was deposited with the European Collection of Animal Cell Cultures (ECACC) under number 1C6 90050301 on May 3, 1990.

As stated before, the beta cell antigen according to the invention has a molecular weight of about 38 kD.

The invention provides the beta cell antigen in the form of an isolated product that can be used directly, for instance for diagnostic uses in which by means of the antigen, autoantibodies or autoreactive T cells in blood from type 1 diabetes patients can be demonstrated. The isolated beta cell antigen (isolated from cells or media containing the antigen) can also be used as a means of obtaining antibodies with specificity for the antigen, using known per se techniques (for instance the known monoclonal antibodies techniques).

The invention further provides a peptide which imitates the epitope of the beta cell antigen according to the invention, said epitope being recognised by T cell clone 1C6, and can be recognised by T cell clone 1C6. Such a peptide can be used instead of the antigen itself in processes for detecting reactive T cells and in processes for generating reactive T cell lines.

The invention also extends to a T cell capable of recognising the beta cell antigen according to the invention as well as to a T cell receptor (TCR) of such a T cell, which TCR is capable of recognizing the beta cell antigen according to the invention. The invention further extends to a peptide which imitates such a TCR capable of recognising the epitope of the beta cell antigen according to the invention recognised by the T cell clone 1C6 and can recognise the epitope of the beta cell antigen according to the invention recognised by the T cell clone 1C6. More in particular, reference is made here to a specific and immunogenic peptide of such a TCR, which can be used for vaccination purposes instead of the T cell (T cell vaccination).

The invention is further incorporated in an antibody having specificity for a T cell capable of recognising the beta cell according to the invention as well as in an antibody having specificity for the beta cell antigen according to the invention.

Finally, the invention alo comprises the use of one of the above materials according to the invention for type 1 diabetes mellitus diagnostics and/or monitoring as well as the use of one of the above materials according to the invention for type 1 diabetes mellitus therapy and/or prevention.

The invention will now be illustrated by an example.

### Example

By homogenising transplantable insulinoma tissue of rats and subjecting it to a discontinuous Nycodenz density gradient centrifugation, four fractions were prepared formed by, respectively, cytosolic constituents, plasma membrane, insulin secretory granules (ISG) and a pellet comprised of a mixture of lysosomes, mitochondria and endoplasmic reticulum as defined by analysis using marker proteins (see Table A). Each fraction was tested for its ability of stimulating the proliferation of a panel of T cell clones isolated from the peripheral blood of a newly diagnosed type 1 diabetes patient. These CD4 expressing, HLA-DR1 restricted T cell clones were generated using a crude membrane preparation from the rat insulinoma cell line RINm5F as antigen. A marked proliferation of the cells in response to the fraction enriched in insulin secretory granules was observed with two clones (1C6 and 1C10) (see Table A). Some reactivity was observed with the pelleted proteins, but the extent of cell proliferation was commensurate with the contamination by insulin secretory granules.

The insulin secretory granule fraction was subjected to Percoll density gradient centrifugation which resulted in a two-fold increase in the specific activity of the granule marker and a two- to five-fold decrease in specific activities of the other marker proteins. The cell proliferation of clones 1C6 and 1C10 in response to these purified granules was not diminished, which indicates that the T cell responses were not attributable to contamination of the granule fraction by a minor cellular constituent.

The ISG preparation was further subfractionated into granule matrix and membrane components. The response of clones 1C6 and 1C10 was confined to the membrane fraction and was retained following a series of extraction steps carried out to remove extrinsic membrane proteins (see Table B).

The molecular weight of the antigen recognised by clone 1C6 was determined using SDS/polyacrylamide gel electrophoresis of the granule membrane followed by T cell proliferation assays with proteins separated for molecular weight by electroelution. The response of this T cell clone was directed against fractions with a mean molecular weight of 38 kD under non-reducing conditions. Reduction of disulphide linkages in the sample before electrophoresis did not affect the apparent size of the antigen.

The cellular distribution of the antigen recognised by clone 1C6 is shown in Table C. In addition to insulin producing tissue, strong positive reactions were observed with pituitary, adrenal and a neuroblastoma cell line. These tissues, together with the pancreatic islets, are members of the diffuse neuroendocrine system and share a number of morphological and molecular features. A common property of the reactive tissues is their storage of bioactive polypeptides in intracellular storage vesicles and secretion by an exocytotic mechanism. They differ in this respect from most of the nonreactive tissues in the tested series. Since tissue from the exocrine pancreas does not elicit cell proliferation, it is unlikely, however, that the antigen is a functional component of the exocytotic machinery.

**TABLE A :**

| subcellular distribution of T cell antigens | | | | | | | |
|---|---|---|---|---|---|---|---|
| fraction | enzymatic activity (nmol/min/mg) | | | | | T cell proliferation (cpm) | |
| | A | B | C | D | E | 1C6 | 1C10 |
| cytosol | 2.8 | 17.1 | 0.0 | 0.5 | 6.7 | 145 ± 71 | 3630 ± 2723 |
| | | | | | | | |
| plasma membranes | 720.7 | 22.1 | 28.8 | 26.3 | 9.0 | 372 ± 138 | 6422 ± 1284 |
| | | | | | | | |
| secretory granules | 91.5 | 27.4 | 135.7 | 27.8 | 24.2 | 23737 ± 2848 | 24537 ± 2944 |
| | | | | | | | |
| pellet | 53.3 | 110.3 | 460.0 | 94.0 | 16.1 | 16513 ± 4128 | 15883 ± 635 |
| A: alkaline phosphatase B: aryl sulphatase C: cytochrome oxidase D: NADPH cytochrome C E: carboxypeptidase H | | | | | | | |

### Legend to Table A

As shown in Table A, T cell clones IC6 and IC10 exhibited a marked proliferation in response to subcellular fractions of insulinoma enriched for the secretory granule marker, carboxypeptidase H. The responses to fractions enriched with cytosol proteins and plasma membrane constituents (marker: alkaline phosphatase) were appreciably lower. The T cell reactivity to the pellet fraction containing endoplasmic reticulum (NADPH cytochrome C reductase), lysosomes (aryl sulphatase) and mitochondria (cytochrome oxidase) could be attributed to a contamination of this fraction by secretory granule components.

### Subcellular fractionation

All steps were carried out at 4°C, unless specified otherwise. Transplantable rat insulinoma tissue (3-5 g) was homogenized in 20 ml 270 mM sucrose, 10 mM MES-KOH, 1 mM EGTA, 1 mM MgSO₄ solution, pH 6.5, and centrifuged for 6 min. at 1770 x g to remove cell debris and nuclei. The supernatant (20 ml) was placed on a three-step (5 ml/step) density gradient formulated by mixing 19.2% (w/v) Nycodenz, 170 mM sucrose, 10 mM MES-KOH solution with the homogenisation media in ratios of 1:3, 1:1 and 1:0. The gradient was centrifuged for 60 min. at 100,000 x g in a Beckman SW28 rotor and the material recovered from the supernatant (cytosol), the 0/4.8% Nycodenz interface (light membranes), the 9.6/19.2% interface (the insulin secretory granules) and the pellet. The particulate fractions were washed twice, by resuspending in homogenisation media and centrifugation for 25 min. with 100,000 x g, and stored at -70°C as 1-2 mg protein/ml in 270 mM MES pH 6.5.

### T cell proliferation

In flat-bottomed 96 well microtitre plates 10⁴ T cells and 5 x 10⁴ irradiated HLA-DR1,1 mononuclear cells as antigen presenting cells were cultured in triplicate in the presence of 5 µg/ml antigen for 3 days. After addition of ³H-thymidine and 16 hours of incubation the cells were harvested on a glass fibre filter. The number of cpm ³H-thymidine incorporation was measured by liquid scintillation counting; in addition to the measured cpm, the standard deviation is also given in the table. Protein determinations were carried out with Pierce BCA reagent, after the membrane samples had first been solubilized by heating for 5 min. at 60°C in 20 µl 0.2% sodium dodecyl sulphate in 10 mM Tris Cl pH 8. The tabulated results, originating from one experiment, are representative of the data obtained in three different assays.

**TABLE B**

| antigen | extraction | T cell proliferation (cpm) | |
|---|---|---|---|
| | | 1C6 | 1C10 |
| homogenate | -- | 43090 ± 1293 | 7517 ± 977 |
| medium | -- | 187 ± 91 | 390 ± 351 |
| | | | |
| pellet 0 | low salt | 33053 ± 2327 | 15587 ± 6235 |
| supernatant 0 | low salt | 2875 ± 690 | 303 ± 151 |
| | | | |
| pellet 1 | high salt | 12592 ± 4064 | 9092 ± 2819 |
| supernatant 1 | high salt | 468 ± 334 | 60 ± 12 |
| | | | |
| pellet 2 | alkaline | 5017 ± 1054 | 1380 ± 138 |
| supernatant 2 | alkaline | 90 ± 17 | 122 ± 31 |

### Legend to Table B

Insulin secretory granules (1-2 mg protein) prepared as described in respect of Table A were extracted in a sequential manner by sonication (20 s, MSE sonifier) in 5 ml of the following ice-cold solutions: 10 mM NH₄HCO₃ (low salt), 10 mM NH₄HCO₃ containing 2 mM EDTA and 1 M NaCl (high salt) or 0.5 M Na₂CO3 (alkaline). After each extraction the material was centrifuged with 240,000 x g for 30 min., and the pellet was washed by resuspension in 5 ml 10 mM NH₄HCO₃ and centrifugation as before. Samples at each step were resuspended in 50 µl 10 mM NH₄HCO₃ and analyzed for protein and antigenic activity as in Table A. The tabulated results, originating from one experiment, are representative of the data obtained in three different assays.

**TABLE C :**

| tissue distribution of the antigen recognised by 1C6 | |
|---|---|
| source of antigen | T cell proliferation (cpm) |
| insulinoma | 31277 ± 4379 |
| neuroblastoma | 31323 ± 1879 |
| pituitary | 10482 ± 3669 |
| adrenal | 23473 ± 1643 |
| medium | 1182 ± 142 |

## Claims

1. A beta cell antigen consisting of an intrinsic transmembrane protein of insulin secretory granules of beta cells lying in pancreatic islets of Langerhans, which protein has a molecular weight of about 38 kD and contains an epitope recognised by T cell clone 1C6 (ECACC 1C6 90050301).

2. A T cell receptor of a T cell capable of recognising the beta cell antigen according to claim 1.

3. A T cell receptor according to claim 2 which is the T cell receptor of T cell clone 1C6.

4. An antibody having specificity for the beta cell antigen according to claim 1.

5. A material according to any one of claims 1-4 for use in type 1 diabetes mellitus diagnostics and/or monitoring.

6. A material according to any one of claims 1-4 for use in type 1 diabetes mellitus therapy and/or prevention.

## Patentansprüche

1. Beta-Zell-Antigen, bestehend aus einem intrinsischen Transmembran-Protein von Insulin sekretierenden Granula von in pankreatischen Langerhansschen Inseln liegenden Beta-Zellen, welches Protein ein Molekulargewicht von ungefähr 38 kD hat und ein von T-Zell-Klon 1C6 (ECACC 1C6 90050301) erkanntes Epitop enthält.

2. T-Zell-Rezeptor einer T-Zelle, der das Beta-Zell-Antigen nach Anspruch 1 erkennen kann.

3. T-Zell-Rezeptor nach Anspruch 2, der der T-Zell-Rezeptor von T-Zell-Klon 1C6 ist.

4. Antikörper mit Spezifizität für das Beta-Zell-Antigen nach Anspruch 1.

5. Material nach einem der Ansprüche 1-4 zur Verwendung bei Typ-1-Diabetes-mellitus-Diagnostik und/oder -Kontrolle.

6. Material nach einem der Ansprüche 1-4 zur Verwendung bei Typ-1-Diabetes-mellitus-Therapie und/oder -Prävention.

## Revendications

1. Un antigène de cellule bêta constitué d'une protéine transmembranaire intrinsèque de granules sécréteurs d'insuline de cellules bêta situées dans les îlots pancréatiques de Langerhans, laquelle protéine possède un poids moléculaire d'environ 38 kD et contient un épitope reconnu par le clone 1C6 de cellule T (ECACC 1C6 90050301).

2. Un récepteur de cellule T d'une cellule T capable de reconnaître l'antigène de cellule bêta conforme à la revendication 1.

3. Un récepteur de cellule T conforme à la revendication 2 qui est le récepteur de cellule T du clone 1C6 de cellule T.

4. Un anticorps ayant une spécificité pour l'antigène de cellule bêta conforme à la revendication 1.

5. Un matériau conforme à l'une quelconque des revendications 1-4 pour emploi dans le diagnostic et/ou la surveillance du diabète mellitus de type 1.

6. Un matériau conforme à l'une quelconque des revendications 1-4 pour emploi dans le traitement et/ou la prévention du diabète mellitus de type 1.
